# EUROPÄISCHE PATENTSCHRIFT

(11) **EP 0 381 717 B1**
(45) Veröffentlichungstag und Bekanntmachung des Hinweises auf die Patenterteilung: **28.04.1993**
(21) Anmeldenummer: 89907652.5
(22) Anmeldetag: 21.07.1989
(51) Int. Cl.: G01N 3/08, G01N 3/30, B65H 63/06

(54) **VERFAHREN ZUR MESSUNG DER GARNFESTIGKEIT**
PROCESS FOR MEASURING YARN STRENGTH
PROCEDE DE MESURE DE LA SOLIDITE DE FILS

(30) Priorität: 03.08.1988 CH 2949/88
(43) Veröffentlichungstag der Anmeldung: 16.08.1990
(73) Patentinhaber: Siegfried Peyer AG, 8832 Wollerau (CH)
(72) Erfinder: TOEDTLI, Sergej, CH-8832 Wollerau (CH)
(74) Vertreter: Lusuardi, Werther Giovanni, Dr.
(86) Internationale Anmeldenummer: CH8900139
(87) Internationale Veröffentlichungsnummer: WO9001689

(56) Entgegenhaltungen:
- EP-A- 0 207 471
- EP-A- 0 241 894
- CH-A- 478 051
- DE-A- 2 152 984
- US-A- 2 123 367
- US-A- 3 370 800
- US-A- 4 173 787

## Beschreibung

Die Erfindung bezieht sich auf ein Verfahren zur Messung der Garnfestigkeit gemäss dem Oberbegriff des Anspruchs 1.

Die möglichst kontinuierliche, zeitverzugslose und ohne grossen Aufwand durchführbare Bestimmung der Garnfestigkeit ist in der Textilindustrie von grosser Bedeutung, da sie zuverlässige Voraussagen über die spätere Eignung des Garnes für vielfältige Verwendungen zulässt. Durch den immer höheren Automatisierungsgrad in der Textilindustrie und die dadurch extrem gesteigerte Verarbeitungsgeschwindigkeit sind die herkömmlichen Messverfahren zur Bestimmung der Garnfestigkeit je länger desto weniger in der Lage zeitlich befriedigende Resultate zu liefern. Oft kann das Resultat der Bestimmung der Garnfestigkeit erst nach bereits erfolgter Verarbeitung zur Kenntnis genommen werden, was allfällige Korrekturen von vorn herein ausschliesst.

Es sind bereits Messverfahren zur Bestimmung der Garnfestigkeit bekannt, beispielsweise aus der US-PS 4.173.787 KATONA ET AL. und der DE-A-2152984 SCHUBERT & SALZER. Ihnen gemeinsam ist der Nachteil dass das Garn "off-line", d.h. im Prüfungslabor, ausserhalb der laufenden Garnverarbeitung geprüft wird und somit keine Korrektur während des Garnverarbeitungsprozesses selbst möglich ist. Bedingt durch die stichprobenweise Messmethodik im Labor wird die Repräsentanz derart ermittelter Daten zunehmends angezweifelt.
Ein weiterer Nachteil der bekannten Messverfahren besteht schliesslich darin, dass damit nur die statische Garnprüfung durchgeführt werden kann, welche für den Garnverarbeiter von untergeordneter Bedeutung ist, weil das Garn in den nachfolgenden Schritten immer nur schlagartig, d.h. dynamisch beansprucht wird.

Hier will die Erfindung Abhilfe schaffen. Der Erfindung liegt die Aufgabe zugrunde, ein Verfahren zur kontinuierlichen, zeitverzugslosen "on-line" Messung der Garnfestigkeit zu schaffen, welches eine lückenlose, flächenhafte Überwachung der Garnproduktion oder Garnverarbeitung mit einem minimalen prüfaufwand pro Garnspindel ermöglicht. Eine weitere Aufgabe besteht in der Schaffung einer Vorrichtung, welche die vorhandene Elektronik in den zum Stand der Technik gehörenden Garnreinigern zur Messung der Garnfestigkeit nach dem Verfahren gemäss der Erfindung mitbenutzt.

Die Erfindung löst die gestellte Aufgabe mit einem Verfahren, welches die Merkmale des Anspruchs 1 aufweist, sowie einer Vorrichtung, welche die Merkmale des Anspruchs 6 aufweist.

Die durch die Erfindung erreichten Vorteile sind im wesentlichen darin zu sehen, dass dank des erfindungsgemässen Verfahrens während des Spinnvorgangs, dem Spul- oder Zwirnprozess eine laufende, lückenlose ("on-line") Ermittlung der Garnfestigkeit ermöglich wird. Durch Nutzung der elektronischen Infrastruktur eines bereits vorhandenen Garnreinigers kann zudem eine apparativ einfache und kostengünstige Konstruktion verwendet werden.

Einige Ausführungsbeispiele der Erfindung, welche zugleich das Funktionsprinzip erläutern, sind in der Zeichnung dargestellt und werden im folgenden näher beschrieben.
Fig. 1 stellt einen Schnitt durch die erfindungsgemässe Vorrichtung bei offener Klemmvorrichtung dar;
Fig. 2 stellt einen Schnitt durch die erfindungsgemässe Vorrichtung dar, bei der die Klemmvorrichtung zusammen mit einem Garnreiniger realisiert ist; und
Fig. 3 stellt einen Schnitt durch die Klemmvorrichtung der erfindungsgemässen Vorrichtung im offenen wie auch im geschlossenen Zustand dar.

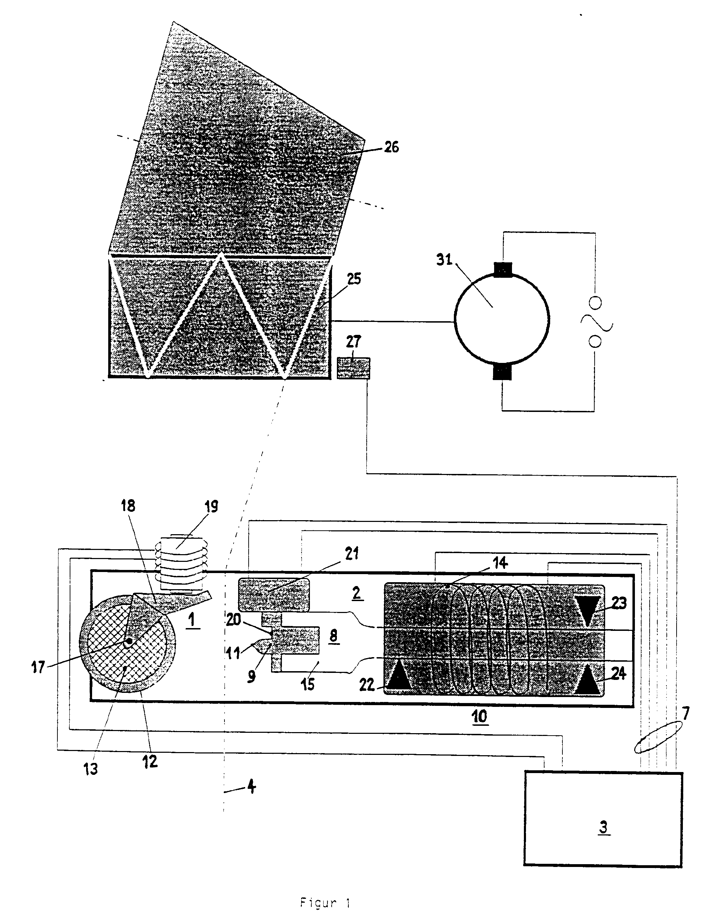

In Fig. 1 ist die bevorzugte Ausführungsform der Vorrichtung zur Durchführung des erfindungsgemässen Verfahrens dargestellt. Die erfindungsgemässe Vorrichtung besteht im wesentlichen aus einer Klemmvorrichtung 1 für das Garn 4, einer Messvorrichtung 2 zur Bestimmung der Garnfestigkeit und einer Zentraleinheit 3 zur Auswertung der ermittelten Messdaten, sowie einer fakultativ vorhandenen Schneidvorrichtung 8 zum Durchschneiden des Garnes 4.

Die Klemmvorrichtung 1 ist in die Schneidvorrichtung 8 integriert, welche ihrerseits aus einem verschiebbar geführten Messer 9 und Antriebsmittel 10, um das Messer 9 mit seiner Schneide 11 gegen eine als Ambossfläche 12 ausgebildete Trommel 13 zu bewegen, besteht. Die Antriebsmittel 10 bestehen im wesentlichen aus dem längsverschiebbar in der elektromagnetischen Spule 14 angeordneten Messerhalter 15.
Weitere Details der Schneidvorrichtung 8 können beispielsweise der CH-PS 478 051 entnommen werden.

Die in die Schneidvorrichtung 8 integrierte Klemmvorrichtung 1 besteht im wesentlichen aus einer drehbar um die Achse 17 der Trommel 13 angeordneten Klappe 18, welche im geöffneter. Betriebszustand, wie er in Fig. 1 dargestellt ist, durch den Hub-Elektromagneten 19 im Ruhezustand festgehalten wird. Soll die Festigkeit des Garnes 4 gemessen werden, wird der Hub-Elektromagnet 19 durch die Zentraleinheit 3 aktiviert, so dass die Klappe 18 in die zu ihr hin bewegte Aussparung 20 des Messers 9 bewegt wird und das Garn 4 in der Aussparung 20 festgeklemmt und dadurch zum Reissen gebracht wird. Die auftretende Reisskraft wird durch den piezoelektrischer. Kraftaufnehmer 21, der mit der Auflagepunkten 22,23,24 zusammenwirkt, in einen elektrischen Impuls umgewandelt, der über die Leitung 7 der Zentraleinheit 3 zur Auswertung zugeführt wird.

Das Garn 4 kann im kontinuierlich fliessenden Zustand festgeklemmt und zum Reissen gebracht werden. Es ist aber auch möglich das anlaufende oder auslaufende Garn 4 festzuklemmen und zum Reissen zu bringen, je nach den Erfordernissen des Garnverarbeiters. Schliesslich gestattet die erfindungsgemässe Vorrichtung auch das Festklemmen und Zerreissen einer Spleissstelle des Garnes 4 um die Garnfestigkeit an dieser schwächsten Stelle zu ermitteln.

Bei einer besonderen Ausführungsform, bei der die erfindungsgemässe Vorrichtung in einem Kreuzspulautomaten oder einer Open-End-Spinnmaschine integriert ist, wird der Klemmvorgang der Klemmvorrichtung 1 mit der Position der durch den Antrieb 31 bewegten Nutentrommel 25 der Kreuzspulmaschine oder Open-End-Spinnmaschine durch den Sensor 27 derart synchronisiert, dass der Klemmvorgang nur bei einer solchen Position der Nutentrommel 25 ausgelöst wird, bei der die Kreuzspule 26 nicht beschädigt wird.

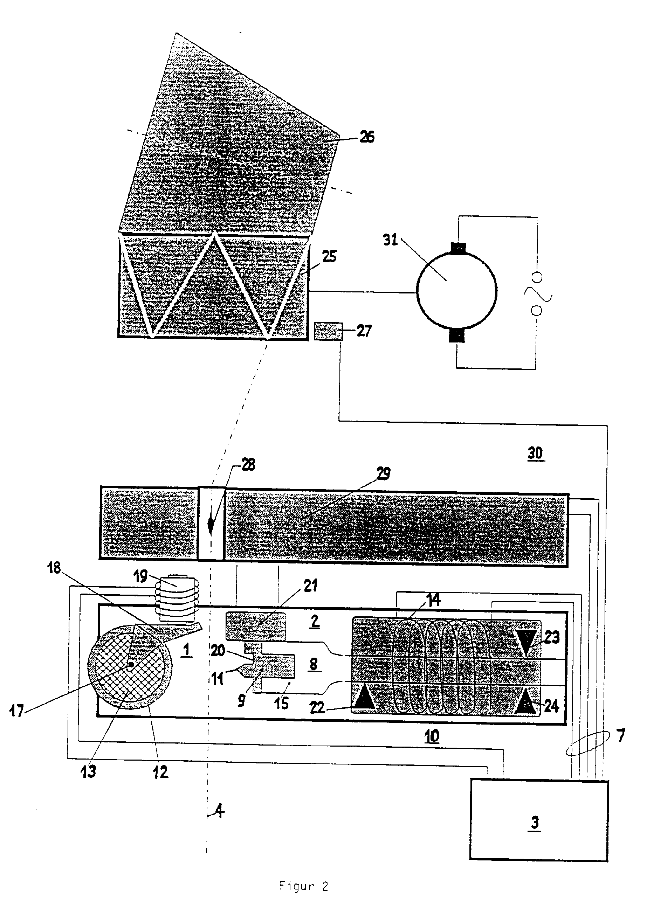

In Fig. 2 ist ein weiteres Ausführungsbeispiel dargestellt, bei dem die Klemmvorrichtung 1 zusammen mit einem Garnreiniger 30 realisiert ist, der aus einem optischen Sensor 29 (mit einer nicht näher im Detail dargestellten Leuchtdiode, dem Messfeld und einem Lichtempfänger), Mittel 7 zur Übertragung und Auswertung der vom optischen Sensor 29 erzeugten Signale, der Zentraleinheit 3 zur Auswertung der elektrischen Signale des optischen Sensors 29 und aus der Schneidvorrichtung 8 besteht. Die Funktion ist nun derart, dass bei der Detektion eines Garnfehlers 28 der Hub-Elektromagnet 19 durch die Zentraleinheit 3 aktiviert wird, so dass die Klappe 18 in die zu ihr hin bewegte Aussparung 20 des Messers 10 bewegt wird und das Garn 4 in der Aussparung 20 festgeklemmt und dadurch zum Reissen gebracht wird. Die auftretende Reisskraft wird durch den piezoelektrischen Kraftaufnehmer 21, der mit den Auflagepunkten 22,23,24 zusammenwirkt, in einen elektrischen Impuls umgewandelt, der über die Leitung 7 der Zentraleinheit 3 zur Auswertung zugeführt wird.
Nach dem Feststellen einer nicht tolerierbaren Abweichung in der Qualität des Garnes 4 im Garnreiniger 30 erfolgt das Festklemmen des laufenden Garnes 4 vorzugsweise rund 1,5 m nach der Festellung des Garnfehlers 28, damit die Reissfestigkeit an einer gesunden Stelle des Garnes 4 ermittelt wird.

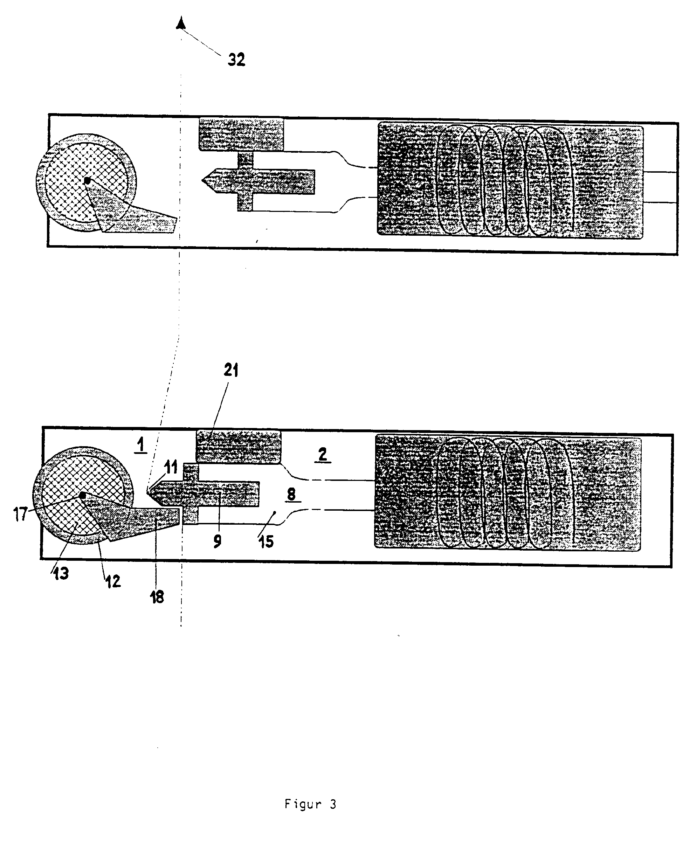

Fig. 3 zeigt im oberen Teil die Klemmvorrichtung 1 der erfindungsgemässen Vorrichtung im offenen Zustand und im unteren Teil im geschlossenen Zustand. Bei diesem Ausführungsbeispiel wird die Klappe 18 - im Gegensatz zum den vorhergehenden Ausführungsbeispielen gemäss Fig. 1 und 2 - unterhalb der Schneidvorrichtung 8 mit einem hier nicht dargestellten Hub-Elektromagneten eingeschwenkt und von der Kraft des in Richtung des Pfeiles 32 laufenden Garnes 4 gegen das Messer 9 festgeklemmt.

## Patentansprüche

1. Verfahren zur on-line Messung der Garnfestigkeit an einer Spinnmaschine oder garnverarbeitenden Maschine, bei dem
A) Garn (4) in einem kontinuierlichen Vorgang durch eine Spinnmaschine oder garnverarbeitenden Maschine bewegt wird,
B) ein Abschnitt des kontinuierlich durch die Maschine laufenden Garnes (4) mittels einer Klemmvorrichtung (1) festgehalten wird,
C) der Abschnitt des Garnes (4) so lange festgehalten wird bis dieser durch die Kraft des bewegten Garnes (4) zum Reissen gebracht wird, und
D) die beim Reissvorgang des festgehaltenen Abschnitts des Garnes (4) auftretenden Kräfte mittels einer Messvorrichtung (2) zur Bestimmung der Garnfestigkeit ermittelt werden.

2. Verfahren nach Anspruch 1, dadurch gekennzeichnet, dass das kontinuierlich anlaufende oder auslaufende Garn (4) festgeklemmt und zum Reissen gebracht wird.

3. Verfahren nach Anspruch 1 oder 2, dadurch gekennzeichnet, dass der Zeitpunkt der Festigkeitsmessung mit der Position einer, der Klemmvorrichtung (1) nachgeschalteten Garntransportvorrichtung, beispielsweise einer Nutentrommel (25) synchronisiert wird.

4. Verfahren nach einem der Ansprüche 1 bis 3, dadurch gekennzeichnet, dass die Messung der Reisskraft unter Benutzung der elektronischen Infrastruktur eines Garnreinigers (30) erfolgt.

5. Verfahren nach einem der Ansprüche 1 bis 4, dadurch gekennzeichnet, dass die Ansteuerung der Klemmvorrichtung (1) durch die Elektronik eines Garnreinigers (30) erfolgt.

6. Vorrichtung zur Durchführung des Verfahrens nach einem der Ansprüche 1 bis 3 an einer Spinnmaschine oder garnverarbeitenden Maschine, wobei die Vorrichtung eine in der Spinnmaschine oder garnverarbeitenden Maschine integrierte Klemmvorrichtung (1) für einen Abschnitt des kontinuierlich durch die Maschine laufenden Garnes (4), eine Messvorrichtung (2) zur Messung der Reisskraft des festgeklemmten Garnes (4) und eine Zentraleinheit (3) zur Steuerung der Klemmvorrichtung (1) sowie zur Auswertung der ermittelten Messdaten und zur Bestimmung der Garnfestigkeit umfasst.

7. Vorrichtung nach Anspruch 6, zur Durchführung des Verfahrens nach Anspruch 4 oder 5, dadurch gekennzeichnet, dass sie zusätzlich einen Garnreiniger (30) umfasst.

8. Vorrichtung nach Anspruch 6 oder 7, dadurch gekennzeichnet, dass sie in eine Kreuzspulmaschine, eine Open-End-Spinnmaschine oder eine Zwirnmaschine eingebaut ist.

9. Vorrichtung nach Anspruch 7 oder 8, dadurch gekennzeichnet, dass der Garnreiniger (30) zusätzlich eine Schneidvorrichtung (8) zum Durchschneiden des Garnes (4) umfasst.

10. Vorrichtung nach Anspruch 9, dadurch gekennzeichnet, dass die Schneidvorrichtung (8) ein verschiebbar geführtes Messer (9) und Antriebsmittel (10), um das Messer (9) mit seiner Schneide (11) gegen eine als Ambossfläche (12) ausgebildete Trommel (13) zu bewegen, umfasst.

11. Vorrichtung nach Anspruch 9 oder 10, dadurch gekennzeichnet, dass die Klemmvorrichtung (1) vom gleichen Antriebsmittel (10) wie die Schneidvorrichtung (8) betätigt wird.

12. Vorrichtung nach einem der Ansprüche 9 bis 11, dadurch gekennzeichnet, dass die Klemmvorrichtung (1), die Schneidvorrichtung (8) und ein Sensor (29) des Garnreinigers (30) mechanisch als Einheit ausgebildet sind.

## Claims

1. A method for on-line measuring of the yarn-strength at a spinning or yarn-processing machine, whereby
A) yarn (4) is moved continuously through a spinning or yarn-processing machine;
B) a section of the yarn (4) continuously moving through the machine is clamped by a clamping device (1);
C) the section of the yarn (4) is being clamped as long as it is made to tear by the force of the moved yarn (4), and
D) the forces arising during the tearing process of the clamped section of the yarn (4) is determined by a measuring device (2) for determining yarn strength.

2. A method according to claim 1, characterized in that the continuously incoming or outgoing yarn (4) is clamped in place and made to tear.

3. A method according to claim 1 or 2, characterized in that the time of strength-measurement is synchronized with the position of a yarn-moving device, for instance a grooved cylinder (25) that is located after the clamping device (1).

4. A method according to one of claims 1 to 3, characterized in that the yarn-strength measurement is carried out using the electronic infrastructure of a yarn-cleaner (30).

5. A method according to one of claims 1 to 4, characterized in that the clamping device (1) is driven by the electronics of a yarn cleaner (30).

6. Device for implementing the method according to one of the claims 1 to 3 at a spinning or yarn-processing machine, whereby the device includes a clamping device (1) for a section of the yarn (4) continuously moving through the machine, a measuring device (2) for determining the yarn strength of the clamped yarn (4) and a central processing unit (3) for controlling the clamping device (1) as well as for analysing the obtained test data and for determining the yarn strength.

7. Device according to claim 6, for implementing the method according to claim 4 or 5, characterized in that it further includes a yarn cleaner (30).

8. Device according claim 6 or 7, characterized in that it is integrated into a cross-wound bobbin winder, an open-end spinning machine or a twisting machine.

9. Device according to claim 7 or 8, characterized in that the yarn cleaner (30) additionally includes a cutter (8) to sever the yarn (4).

10. Device according to claim 9, characterized in that the cutter (8) comprises a displaceably guided knife (9) and drive means (10) in order to move the knife (9) by its blade (11) toward a drum (13) serving as an anvil surface (12).

11. Device according to claim 9 or 10, characterized in that the clamping device (1) is actuated by the same drive means (10) as the cutter (8).

12. Device according to one of the claims 9 to 11, characterized in that the clamping device (1), the cutter (8) and a sensor (29) of the yarn cleaner (30) form one mechanical unit.

## Revendications

1. Procédé de mesure en ligne de la résistance de fils dans une machine à filer ou une machine à traiter des fils, dans lequel:
A) un fil (4) est déplacé de manière continue par une machine à filer ou une machine à traiter des fils,
B) une section du fil (4) traversant en continu la machine est immobilisée par un dispositif d'immobilisation (1),
C) La section de fil (4) est immobilisée jusqu'à ce que ce fil se déchire sous l'action de la force du fil (4) en mouvement, et
D) les forces apparaissant lors de la déchirure de la section immobilisée du fil (4) sont mesurées par un dispositif de mesure (2) pour déterminer la résistance du fil.

2. Procédé selon la revendication 1, caractérisé en ce que le fil (4) entrant ou sortant en continu est immobilisé et amené à se déchirer.

3. Procédé selon la revendication 1 ou 2, caractérisé en ce que le moment de la mesure de la résistance est synchronisé sur la position d'un dispositif de transport du fil, par exemple un tambour à rainures (25), installé en aval du dispositif d'immobilisation (1).

4. Procédé selon l'une des revendications 1 à 3, caractérisé en ce que la mesure de la force de déchirure s'effectue en recourant à l'infrastructure électronique d'un dispositif de nettoyage (30) du fil.

5. Procédé selon l'une des revendications 1 à 4, caractérisé en ce que la commande du dispositif d'immobilisation (1) est effectuée par l'électronique d'un dispositif de nettoyage (30) du fil.

6. Dispositif destiné à mettre en oeuvre le procédé selon l'une des revendications 1 à 3 sur une machine à filer ou une machine de traitement de fil, le dispositif comprenant un dispositif d'immobilisation (1) d'une section du fil (4) traversant la machine en continu, intégré à la machine à filer ou à la machine de traitement du fil, un dispositif de mesure (2) destiné à mesurer la force de déchirure du fils (4) immobilisé et une unité centrale (3) destinée à commander le dispositif d'immobilisation (1) ainsi qu'à évaluer les données de mesure et à déterminer la résistance du fil.

7. Dispositif selon la revendication 6, destiné à exécuter le procédé selon la revendication 4 ou 5, caractérisé en ce qu'il comprend en supplément un dispositif de nettoyage (30) du fil.

8. Dispositif selon la revendication 6 ou 7, caractérisé en ce qu'il est incorporé dans une machine de bobinage croisé, une machine à filer ouverte ou une machine à tourbillonner.

9. Dispositif selon la revendication 7 ou 8, caractérisé en ce que le dispositif de nettoyage (30) du fil comprend en supplément un dispositif de découpe (8) servant à couper le fil (4).

10. Dispositif selon la revendication 9, caractérisé en ce que le dispositif de découpe (8) comprend un couteau (9) à déplacement guidé et des moyens d'entraînement (10) servant à déplacer le tranchant (11) du couteau (9) contre un tambour (13) configuré en forme de surface d'enclume (12).

11. Dispositif selon la revendication 9 ou 10, caractérisé en ce que le dispositif d'immobilisation (1) est actionné par les mêmes moyens d'entraînement que le dispositif de découpe (8).

12. Dispositif selon l'une des revendications 9 à 11, caractérisé en ce que le dispositif d'immobilisation (1), le dispositif de découpe (8) et un détecteur (29) du dispositif de nettoyage (30) du fil sont réalisés sous la forme d'une unité mécanique intégrée.
